# EUROPÄISCHE PATENTSCHRIFT

(11) **EP 3 330 247 B1**
(45) Veröffentlichungstag und Bekanntmachung des Hinweises auf die Patenterteilung: **08.05.2019**
(21) Anmeldenummer: 16201929.3
(22) Anmeldetag: 02.12.2016
(51) Int. Cl.: C07C 45/28, C07C 49/603

(54) **KATALYTISCHE OXIDATION VON 3,5,5-TRIMETHYLCYCLOHEXA-3-EN-1-ON (BETA-ISOPHORON) MIT WASSERSTOFFPEROXID ZU 2,6,6-TRIMETHYL-2-CYCLOHEXENE-1,4-DION (KETO-ISOPHORON)**
CATALYTIC OXIDATION OF 3,5,5-TRIMETHYLCYCLOHEXA-3-ENE-1-ONE (BETA-ISOPHORONE) WITH HYDROGEN PEROXIDE TO 2,6,6-TRIMETHYL-2-CYCLOHEXENE-1,4-DION (KETO ISOPHORONE)
OXYDATION CATALYTIQUE DE 3,5,5-TRIMÉTHYL-CYCLOHEXA-3-ÈNE-1-ONE (BÊTA-ISOPHORONE) AVEC DU PEROXYDE D'HYDROGÈNE POUR FORMER DU 2,6,6-TRIMÉTHYL-2-CYCLOHEXÈNE -1,4-DIONE (CETO-ISOPHORONE)

(43) Veröffentlichungstag der Anmeldung: 06.06.2018
(73) Patentinhaber: Evonik Degussa GmbH, 45128 Essen (DE)
(72) Erfinder: BAJUS, Stephanie, 63450 Hanau (DE); CASSENS, Jan, 45665 Recklinghausen (DE); DÖRING, Jens, 44141 Dortmund (DE); NITZ, Jörg-Joachim, 45257 Essen (DE); KOHLSTRUCK, Stephan, 45966 Gladbeck (DE); JANSEN, Robert, 46244 Bottrop (DE)

(56) Entgegenhaltungen:
- WO-A1-2015/177011
- DE-A1- 19 619 570
- US-A1- 2002 025 906

## Beschreibung

Gegenstand der Erfindung ist ein neuartiges Verfahren zur Herstellung von 2,6,6-Trimethyl-2-cyclohexen-1,4-dion (Keto-Isophoron) durch katalytische Oxidation von 3,5,5-Trimethylcyclohexa-3-en-1-on (β-Isophoron) mit Wasserstoffperoxid als Oxidationsmittel.

### Stand der Technik

Die vorliegende Erfindung betrifft die Epoxidierung von 3,5,5-Trimethylcyclohexa-3-en-1-on (β-Isophoron, β-IP) und die direkte Weiterreaktion dieser Verbindung zu 2,6,6-Trimethyl-2-cyclohexen-1,4-dion (Keto-Isophoron) mittels eines Katalysatorsystems bestehend aus einem Wolframsalz (Natriumwolframat), Phosphorsäure und einem Phasentransferreagenz in einer Eintopfsynthese.

Isophoron (IP) wird durch Kondensation von Aceton gewonnen es handelt sich um das trimere Kondensationsprodukt. Nach der Reaktion liegt ein Gemisch der Isomeren 3,5,5-Trimethylcyclohexa-2-en-1-on (α-Isophoron) und 3,5,5-Trimethylcyclohexa-3-en-1-on (β-Isophoron) vor vor (US8889914 B2)

Durch Isomerisierung von 3,5,5-Trimethylcyclohexa-2-en-1-on (α-Isophoron) in der Flüssigphase in Gegenwart eines homogenen oder heterogenen Katalysators (DE 19639569 A1) lässt sich 3,5,5-Trimethylcyclohexa-3-en-1-on (β-Isophoron) in sehr guten Ausbeuten herstellen.

Basierend auf β-IP lässt sich Trimethylhydrochinondiacetat (TMHQ-DA) herstellen, welches als Vorstufe zur Herstellung von Vitamin E verwendet werden kann. Somit erlaubt IP Zugang zu Vitamin E und kann in diesem Zusammenhang als Alternative zu Trimethylphenol betrachtet werden, welches ebenfalls eine Vorstufe von Vitamin E darstellt.

Ein wichtiger und für bisherige Verfahren die Wirtschaftlichkeit bestimmender Schritt zur Herstellung von Vitamin E basierend auf Isophoron (IP) ist die Umwandlung zu der Zwischenkomponente Keto-Isophoron.

Aus der Literatur ist die Oxidation von β-Isophoron mittels Peroxycarbonsäuren zum Epoxy-Isophoron bekannt (DE 10024265/US6469215). In den Patentbeispielen wird neben der Oxidation von β-Isophoron auch die Herstellung von Perpropionsäure aus Propionsäure und Wasserstoffperoxid und die Umsetzung von Epoxy-Isophoron zu Dihydroketo-Isophoron beschrieben.

Die hohe Anzahl von Reaktionsschritten und Verfahrensschritten bis hin zum Keto-Isophoron und die Verwendung von sicherheitskritischen Peroxycarbonsäuren und teuren Katalysatorsystemen ist allerdings sowohl aus ökonomischen, als auch aus Sicherheitsgründen ein großer Nachteil dieser Methode.

Daneben ist die Direktoxidation von β-Isophoron zum Keto-Isophoron bekannt (DE 19619570). Dabei wird das β-Isophoron mit Sauerstoff und mit Hilfe eines teuren Mangan-Salen-Katalysators bei erhöhtem Druck (bis 10 bar) oxidiert. Der erhöhte Druck und die Notwendigkeit der Verwendung von reinem Sauerstoff stellen dabei eine sicherheitstechnische Herausforderung für den Bau von Produktionsanlagen dar.

Aus wissenschaftlichen Veröffentlichungen ist auch die Direktoxidation von α-Isophoron direkt zu Ketoisophoron beschrieben (1.) *Catalysis Commun 11_2010_758-762*/2.) *Applied Catalysis A General_2008_104-111.* Allerdings sind die Umsätze & Selektivitäten zu Keto-Isophoron zu gering, die verwendeten Katalysatoren und/oder Oxidationsmittel (z. B.: organische Peoxoverbindungen oder N-hydroxy phthalimide (NHPI)) zu teuer, um Keto-Isophoron großtechnisch herzustellen.

Aufgabe war es, ein Verfahren zu finden, dass die Nachteile, wie oben beschrieben, nicht aufweist, und damit eine einfachere Methode zur Herstellung von Keto-Isophoron zu finden.

Überraschenderweise wurde gefunden, dass der direkte Umsatz von β-Isophoron zu Keto-Isoporon in hohen Umsätzen und Ausbeuten mit Hilfe von Wolfram-Polyoxometallat, sogenannten (POM)-Katalysatoren als aktiver Katalysator, unter Verwendung von Wasserstoffperoxid als kostengünstiges Oxidationsmittel, erreicht werden kann.

Es wurde gefunden, dass das Keto-Isophoron direkt in einer mit Wolfram-POM-katalysierten "One pot"-Oxidationsreaktion aus β-Isophoron gebildet werden kann.

Gegenstand der Erfindung ist ein Verfahren zur Herstellung von Keto-Isophoron durch katalytische Oxidation von β-Isophoron in Gegenwart mindestens eines Katalysators und Wasserstoffperoxid in einer Eintopfsynthese, wobei die Oxidation in Gegenwart von mindestens einem Phasentransferreagenz in einem Zweiphasensystem erfolgt, bestehend aus
A) mindestens einer organischen Phase
   und
B) mindestens einer wässrigen Phase,
   wobei das Zweiphasensystem enthält:
   1) mindestens ein Wolfram-Polyoxometallat als Katalysator und Wasserstoffperoxid, und/oder
   2) eine Mischung aus
      a) mindestens einer Mineralsäure,
      b) Wasserstoffperoxid,
      c) mindestens einem Metallwolframat.

Die wässrige Phase beinhaltet Wasserstoffperoxid als Oxidationsmittel. Das Wolfram-POM-Katalysatorsystem kann während der Reaktion *in situ* generiert (gemäß 2) oder im Voraus gebildet (gemäß 1) eingesetzt werden. Dabei reagiert das Metallwolframat mit der Mineralsäure, bevorzugt Phosphorsäure, und Wasserstoffperoxid in wässriger Phase zum Wolfram-Polyoxometallat (POM).

Der Katalysator Wolfram-POM wird hergestellt aus mindestens einer Mineralsäure, bevorzugt aus Phosphorsäure, mindestens einem Metall-Wolframat, bevorzugt aus dem Metall Natrium, wobei dann in Gegenwart von Wasserstoffperoxid die Bildung von Wolfram-Polyoxometallat (Wolfram-POM) erfolgt.

Wolfram-Polyoxometallat (Wolfram-POM) Katalysatoren können wie auch in WO2015177011 A1 beschrieben hergestellt werden:
Als Ausgangsmaterial für die Bildung der katalytisch aktiven Übergangsmetallverbindung eignen sich insbesondere Derivate von Wolfram. Als Derivate kommt vor allem ein Oxid, ein gemischtes Oxid, eine sauerstoffhaltige Säure, ein Salz einer sauerstoffhaltigen Säure, ein Carbonylderivat, ein Sulfid, ein Chlorid, ein Oxichlorid oder ein Stearat des Elements Wolfram in Betracht.

Geeignete Derivate sind beispielsweise die Metallcarbonyle W(CO)₆, die Oxide WO₂, W₂O₅, WO₃, die Sulfide WS₂ oder WS₃. Weitere Beispiele sind die Sauerstoffsäuren H₂WO₄ und H₂MoO₄ bzw. deren Alkali- oder Erdalkalisalze. Besonders geeignet sind insbesondere Wolframsäuresalze, insbesondere Natrium- oder Kaliumwolframat. Als besonders geeignete Verbindung zur Bildung der katalytisch aktiven Übergangsmetallverbindung in situ hat sich Natriumwolframat, Na₂WO₄, erwiesen.

Die Bildung der katalytisch aktiven Übergangsmetallverbindung in situ erfolgt bevorzugt durch Umsetzung mit einer Mineralsäure von Phosphor. Die Bildung der katalytisch aktiven Übergangsmetallverbindung kann allerdings auch in einer getrennten Reaktion geschehen, wobei zunächst eine oben beschriebene Übergangsmetallverbindung mit einer Mineralsäure von Phosphor und/oder Arsen sowie Wasserstoffperoxid umgesetzt wird und die daraus erhaltene katalytisch aktive Übergangsmetallverbindung dem Zwei-Phasensystem der Epoxidierungsreaktion zugesetzt wird.

Die Mineralsäure von Phosphor wird dem Zwei-Phasensystem zugesetzt. Dabei entsteht eine katalytisch aktive Übergangsmetallverbindung, die sowohl das Übergangsmetall als auch Phosphor umfasst.

Besonders wirksame Mineralsäuren von Phosphor sind Phosphorsäure, phosphorige Säure, Polyphosphorsäure, Pyrophosphorsäure.

In einer besonders bevorzugten Ausführungsform wird die katalytisch aktive Übergangsmetallverbindung, der Katalysator, in situ durch Umsetzung eines Wolframsäuresalzes, insbesondere Natrium-, Kalium- oder Ammoniumwolframat, mit Phosphorsäure und mit Wasserstoffperoxid gebildet.

Bevorzugt liegt das Stoffmengenverhältnis der Phosphorsäure zum Übergangsmetall im Bereich von 0,1 bis 10,0 besonders bevorzugt von 0,25 bis 5,0 am meisten bevorzugt von 1 bis 2.

Das hergestellte Katalysatorsystem kann in einem breiten Temperaturbereich von 1°C - 200°C eingesetzt werden

Aufgrund der schlechten Löslichkeit von β-Isophoron in Wasser wird ein Phasentransferreagenz benötigt. Das Phasentransferreagenz ermöglicht den Übergang der durch Oxidation mit Wasserstoffperoxid gebildeten oxidationsaktiven Katalysatorspezies Wolfram-POM in die organische Phase. Dort findet die Oxidation des β-IP zum Keto-Isophoron statt. Anschließend wird der reduzierte Katalysator in der wässrigen Phase durch das Wasserstoffperoxid zum Wolfram-POM reoxidert.

Das Phasentransferreagenz umfasst ein Kation oder eine Verbindung, die in der wässrigen Phase ein Kation bildet, wobei das Kation mit einem Peroxowolframat oder Heteropolyperoxowolframat ein in der organischen Phase lösliches Salz bilden kann. Vorzugsweise umfasst das Phasentransferreagenz ein einfach geladenes Kation oder eine Verbindung, die in der wässrigen Phase ein einfach geladenes Kation bildet.

Als Phasentransferkatalysator eignen sich quaternäre Ammoniumsalze, tertiäre Amine oder quaternäre Phosphoniumsalze.

Geeignete quaternäre Ammoniumsalze sind Tetraalkylammoniumsalze mit insgesamt mindestens 12 Kohlenstoffatomen in den Alkylgruppen, beispielsweise Dodecyltrimethylammoniumsalze, Hexadecyltrimethyl-ammoniumsalze, Octadecyltrimethyl-ammoniumsalze, Methyltributylammoniumsalze und Methyltrioctylammoniumsalze. Geeignet sind auch quaternäre Ammoniumsalze mit ein- oder zweiwertigen Anionen, beispielsweise Chlorid, Bromid, Nitrat, Sulfat, Hydrogenphosphat, Dihydrogenphosphat, Methylsulfonat, Methylsulfat und Ethylsulfat.

Geeignete tertiäre Amine sind Dodecyldimethylamin, Hexadecyldimethylamin, Octadecyldimethylamin, Tributylamin und Trioctylamin.

Geeignete quaternäre Phosphoniumsalze sind Alkyl-, Benzyl und Phenylphosphoniumsalze, dieses sind zum Beispiel Di-tert-butylmetylphosphomium tetrafluoroborat, Benzyltriphenylphosphoniumchlorid und Triphenylbutylphoniumchlorid.

Das bevorzugt verwendete Katalysatorsystem besteht aus Natriumwolframat, Phosphorsäure, sowie einem Phasentransferreagenz, bevorzugt Trioctylamin welches unter Reaktionsbedingungen zum entsprechenden Ammoniumsalz protoniert wird.

Die Oxidation des β-Isophorons zum Keto-Isophoron läuft dabei in mehreren Schritten ab:
1. Epoxidation des β--Isophorons (A) zum Epoxid (B),
2. Umlagerung des Epoxids (B) zum Hydroxy-Isophoron, (HIP), (C),
3. Oxidation des HIP zum Keto-Isophoron, (KIP), (D).

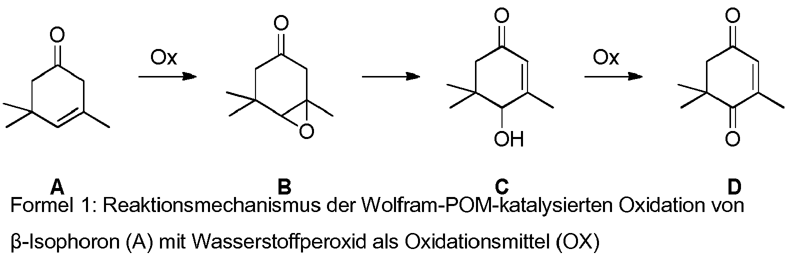

Die Oxidation des β-Isophorons zum Keto-Isophoron kann unter den folgenden Bedingungen erfolgen:
Die Temperatur variiert von 30 - 120 °C, bevorzugt im Bereich zwischen 50-80°C.

Das Verhältnis β-Isophoron zu H₂O₂ beträgt 1:0,7 - 1:3 Gew.-%, bevorzugt 1:1 - 1:2,2 Gew.-%.

Das Verhältnis organische Phase (insbesondere β-Isophorons, HIP, KIP) zu wässriger Phase (insbesondere Wasser & H₂O₂) variiert von 1:10 bis 5:1 Gew.-%, bevorzugt 1:4 - 2:1 Gew.-%.

Die H₂O₂ Konzentration in der wässrigen Phase beträgt 1 -50 Gew.-%, bevorzugt 2-30 Gew.-%.

Das molare Verhältnis Katalysator (Wolfram POM) zu H₂O₂ beträgt 1:10 - 1:400, bevorzugt 1:50 - 1:200.

### Beispiele:

### Allgemeine Versuchsdurchführung der Beispiele (erfindungsgemäß)

In einem 50 ml Dreihals-Rundkolben mit Rückflusskühler und Rührer wird ein Gemisch aus Na₂WO₄ x 2 H₂O, H₃PO₄, Phasentransferreagenz (PTC), β-Isophoron, und Wasser vorgelegt und auf Reaktionstemperatur (T) erhitzt. Nach Erreichen der Reaktionstemperatur wird die notwendige Menge des 30%iges Wasserstoffperoxids innerhalb von 5 Minuten mit Hilfe einer Spritze in den Kolben dosiert. Anschließend wir das Reaktionsgemisch für die restliche Reaktionszeit gerührt. Nach Versuchsende wird das Gemisch unter ständigem Rühren abgekühlt und mit Dioxan homogenisiert. Anschließend wird das Gemisch per Gaschromatographie, NMR (Bestimmung der organischen Verbindungen) und Cerimetrie (H₂O₂-Analytik) analysiert.

Ziel war es dabei, einen möglichst hohen Umsatz (U) an β-Isophoron und gleichzeitig eine hohe Ausbeute (A) an Oxidationsprodukten (HIP + KIP) zu erzielen. Daneben spielt auch der H₂O₂-Nutzungsgrad (Quotient aus der Summe der Stoffmengen KIP + HIP und eingesetzter Stoffmenge H₂O₂) eine wichtige Rolle für einen effizienten Prozess. Als Einflussfaktoren wurden variert:
- Phasentransferreagenz (Beispiele 1 - 4)
- Temperatur (Beispiele 4 - 6)
- H₂O₂-Menge (Beispiel 4 & 7)

### Reaktionsbedingungen:

50 ml Rundkolben, 2,5 g β-Isophoron (18 mmol), 2,25- 4,5 g 30%ige H₂O₂-Lösung (wässrig, 40 mmol), Wolfram-POM-Katalysator (100 mg Na₂WO₄ x 2 H₂O (0,3 mmol), 200 mg H₃PO₄(85%ig, 1,7 mmol), 0,23 mmol), Phasentransferreagenz (1. Keine / 2. Rewoquat CR 3099 / 3. Trioctylmethylammonium-methylsulfat (TMAMS), 4. Trioctylamin), 5 g Wasser, pH = 2, Dauer = 2 h, Temperatur 70-90°C. Die jeweiligen Reaktionsbedingungen der Beispiele 1-9 der Wolfram-Polyoxometallat-katalysierten Oxidation von β-Isophoron mit Wasserstoffperoxid sind in Tabelle 1 zusammengefasst

### Beispiele 1 - 4 (Variation Phasentransferreagenz)

Die Ergebnisse mit unterschiedlichen Phasentransferreagenzien sind in Tabelle 1 (Eintrag 1 bis 4) dargestellt. Die Umsetzung des Isophorons zum Keto-Isophoron stellt eine zweistufige Oxidation dar, weshalb ein 2,2 facher Überschuss an H₂O₂ bezogen auf β-Isophoron eingesetzt wurde, die Reaktionstemperatur (T) betrug 80°C.

Ohne Phasentransferreagenz läuft die Zielreaktion nur sehr langsam ab. Das β-Isophoron wird vorwiegend zu α-Isophoron isomerisiert und zu unerwünschten Nebenprodukten umgesetzt (Tabelle 1 Eintrag 1).

Durch den Einsatz von Trioctylamin als PTC, welches unter Reaktionsbedingungen zum ensprechenden Ammoniumsalz protoniert wird, konnte die höchste Ausbeute an Keto-Isophoron erzielt werden (Tabelle 1 Eintrag 4). Der Umsatz von β-Isophoron war nahezu vollständig (U = 99%), und die Ausbeute (A) an Keto-IP lag bei 42%. Aber auch α-Isophoron (Ausbeute: 10%) und insbesondere Hydroxy-Isophoron (Ausbeute 35%) stellt einen Wertstoff dar, da diese in einem kontinuierlichen Produktionsverfahren vom restlichen Reaktionsgemisch abgetrennt und wieder in den Herstellungsprozess zurückgeführt werden können. α-Isophoron kann wieder zu β -Isophoron isomerisiert werden und HIP kann direkt in die Reaktion zurückgefahren werden um weiter zum Keto-IP oxidiert zu werden. Somit liegt die Ausbeute der Summe der oxidierten Moleküle (HIP und KIP) liegt dann bei 77%. Die Ausbeute an Wertstoffen (α-Isophoron, HIP und KIP) liegt sogar bei 87%.

### Beispiele 4 - 6 (Variation Temperatur)

Die Ergebnisse bei unterschiedlichen Temperaturen (70-90°C) sind in Tabelle 1 (Eintrag 4 bis 6) dargestellt. Die Umsetzung des Isophorons zum Keto-Isophoron wurde wiederum bei einem 2,2 fachen Überschuss an H₂O₂ bezogen auf β-Isophoron durchgeführt, als Phasentransferreagenz wurde Trioctylamin eingesetzt.

Eine Reaktionstemperatur von 70 °C führte zu einer geringeren KIP Ausbeute (24%), aber zu einer erhöhten HIP-Ausbeute (67%). Die Ausbeute der Summe der oxidierten Moleküle (HIP und KIP) liegt dann bei 91% und ist um 14% höher als bei einer Reaktionstemperatur von 80°C, auch der H₂O₂-Nutzungsgrad steigt leicht auf 53% an.

Bei 90 °C Reaktionstemperatur werden hingegen vermehrt unerwünschte Neben/Folgeprodukte gebildet, die Summe aus KIP- und HIP-Ausbeute sinkt auf 72% und auch der H₂O₂-Nutzungsgrad sinkt leicht auf 48%.

### Beispiele 4 & 7 (Variation H₂O₂-Menge)

Der H₂O₂-Nutzungsgrad kann durch die Verringerung der eingesetzten H₂O₂-Menge deutlich erhöht werden. Die Differenz zwischen H₂O₂-Umsatz und H₂O₂-Nutzung kann durch Zersetzung und die Bildung von Neben/Folgeprodukte erklärt werden, dieses gilt für alle Beispiele (Beispiele 1-7).

Verringert man die eingesetzte H₂O₂-Menge um 50% auf die 1,1 fache Isophoron-Stoffmenge, so erhöht sich der H₂O₂-Nutzungsgrad auf zu 85%! Die Summe der oxidierten Moleküle (HIP und KIP) liegt aber weiterhin bei sehr guten 76%.

### Fazit:

2,6,6-Trimethyl-2-cyclohexen-1,4-dion (Keto-Isophoron) lässt sich durch katalytische Oxidation von 3,5,5-Trimethylcyclohexa-3-en-1-on (beta-Isophoron) mit Wasserstoffperoxid als Oxidationsmittel effizient in einer Eintopfsysnthese herstellen. Neben Keto-Isophoron (KIP) bildet sich auch Hydroxy-Isophoron (HIP), welches in einem kontinuierlichen Prozess vom restlichen Reaktionsgemisch abgetrennt und weiter zu Keto-Isophoron oxidiert werden kann.

Mit dem verwendeten Katalysatorsystem bestehend aus Natriumwolframat, Phosphorsäure, sowie Trioctylamin als Phasentransferreagenz wurde eine Ausbeute an oxidierten Molekülen (HIP und KIP) von bis zu 91% erreicht. Neben den sehr guten Ausbeuten an Wertprodukten (HIP + KIP) auch der H₂O₂-Nutzungsgrad von bis zu 85% exzellent.

**Tabelle 1**

| Eintrag | PTC | m β - IP | m H₂O₂ (30%ig) | T | U (β-IP) | A (α-IP) | A (KIP) | A (HIP) | U (H₂O₂) | H₂O₂-Nutzung |
|---|---|---|---|---|---|---|---|---|---|---|
| | | [g] | [g] | [°C] | [%] | [%] | [%] | [%] | [%] | [%] |
| 1 | - | 2,5 | 4,5 | 80 | 65 | 22 | 5 | 13 | 31 | 10 |
| 2 | Rewoquat | 2,5 | 4,5 | 80 | 98 | 8 | 24 | 52 | 90 | 46 |
| 3 | TMAMS | 2,5 | 4,5 | 80 | 97 | 8 | 29 | 24 | 72 | 38 |
| 4 | Trioctylamin | 2,5 | 4,5 | 80 | 98 | 10 | 42 | 35 | 80 | 51 |
| 5 | Trioctylamin | 2,5 | 4,5 | 70 | 99 | 8 | 24 | 67 | 86 | 53 |
| 6 | Trioctylamin | 2,5 | 4,5 | 90 | 99 | 15 | 46 | 16 | 90 | 48 |
| 7 | Trioctylamin | 2,5 | 2,25 | 80 | 95 | 13 | 15 | 61 | 98 | 85 |

| | | | | | | | | | | |
|---|---|---|---|---|---|---|---|---|---|---|
| 50 ml Rundkolben, 2,5 g beta-Isophoron (18 mmol), 2,25-4,5 g 30%ige H₂O₂-Lösung (wässrig, 20-40 mmol), 100 mg Na₂WO₄ x 2 H₂O (0,3 mmol), 200 mg H₃PO₄ (85%ig, 1,7 mmol), 0,23 mmol Phasentransferreagenz (PTC), 5 g Wasser, pH -Bereich = 1,5-2, 2 h. | | | | | | | | | | |

## Patentansprüche

1. Verfahren zur Herstellung von Keto-Isophoron durch katalytische Oxidation von β-Isophoron in Gegenwart mindestens eines Katalysators und Wasserstoffperoxid in einer Eintopfsynthese, wobei die Oxidation in Gegenwart von mindestens einem Phasentransferreagenz in einem Zweiphasensystem erfolgt, bestehend aus
A) mindestens einer organischen Phase
und
B) mindestens einer wässrigen Phase,
wobei das Zweiphasensystem enthält:
1) mindestens ein Wolfram-Polyoxometallat als Katalysator und Wasserstoffperoxid,
und/oder
2) eine Mischung aus
a) mindestens einer Mineralsäure,
b) Wasserstoffperoxid,
c) mindestens einem Metallwolframat.

2. Verfahren nach Anspruch 1,
**dadurch gekennzeichnet, dass** die Oxidation bei Temperaturen von 30 - 120 °C, bevorzugt im Bereich zwischen 50-80°C, durchgeführt wird.

3. Verfahren nach mindestens einem der vorherigen Ansprüche,
**dadurch gekennzeichnet, dass** das Verhältnis von β-Isophoron zu H₂O₂ 1:0,7 - 1:3 Gew.-%, bevorzugt 1:1 - 1:2,2 Gew.-%, beträgt.

4. Verfahren nach mindestens einem der vorherigen Ansprüche,
**dadurch gekennzeichnet, dass** das Verhältnis organische Phase zu wässriger Phase von 1:10 bis 5:1 Gew.-%, variiert, bevorzugt 1:4 - 2:1 Gew.-%.

5. Verfahren nach mindestens einem der vorherigen Ansprüche,
**dadurch gekennzeichnet, dass** die H₂O₂ Konzentration in der wässrigen Phase 1 -50 Gew.-%, bevorzugt 2-30 Gew.-% beträgt.

6. Verfahren nach mindestens einem der vorherigen Ansprüche,
**dadurch gekennzeichnet, dass** das molare Verhältnis von Katalysator zu H₂O₂ 1:10 - 1:400 Gew.-%, bevorzugt 1:50 - 1:200 Gew.-%, beträgt.

7. Verfahren nach mindestens einem der vorherigen Ansprüche,
**dadurch gekennzeichnet, dass** als Phasentransferreagenz quaternäre Ammoniumsalze, tertiäre Amine oder quaternäre Phosphoniumsalze, allein oder in Mischungen, eingesetzt werden.

8. Verfahren nach Anspruch 7,
**dadurch gekennzeichnet, dass** als Phasentransferreagenz Dodecyltrimethylammoniumsalze, Hexadecyltrimethyl-ammoniumsalze, Octadecyltrimethylammoniumsalze, Methyltributylammoniumsalze, Methyltrioctylammoniumsalze, Dodecyldimethylamin, Hexadecyldimethylamin, Octadecyldimethylamin, Tributylamin, Trioctylamin, Alkyl-, Benzyl- und Phenylphosphoniumsalze, bevorzugt Di-tert-butylmetylphosphomium tetrafluoroborat, Benzyl-triphenylphosphoniumchlorid und Triphenylbutylphoniumchlorid, allein oder in Mischungen, eingesetzt werden

9. Verfahren nach mindestens einem der vorherigen Ansprüche,
**dadurch gekennzeichnet, dass** als Mineralsäuren Phosphorsäure, phosphorige Säure, Polyphosphorsäure, Pyrophosphorsäure, allein oder in Mischungen, eingesetzt werden.

10. Verfahren nach mindestens einem der vorherigen Ansprüche,
**dadurch gekennzeichnet, dass** Natrium- oder Kaliumwolframat, allein oder in Mischungen, eingesetzt wird.

11. Verfahren nach mindestens einem der vorherigen Ansprüche,
**dadurch gekennzeichnet, dass** das verwendete Katalysatorsystem aus Natriumwolframat, Phosphorsäure, sowie einem Phasentransferreagenz besteht.

12. Verfahren nach mindestens einem der vorherigen Ansprüche,
**dadurch gekennzeichnet, dass** als Phasentransferreagenz Trioctylamin eingesetzt wird.

## Claims

1. Process for producing keto-isophorone by catalytic oxidation of β-isophorone in the presence of at least one catalyst and hydrogen peroxide in a one-pot synthesis, wherein the oxidation is effected in the presence of at least one phase transfer reagent in a biphasic system consisting of
A) at least one organic phase
and
B) at least one aqueous phase
wherein the biphasic system comprises:
1) at least one tungsten polyoxyometallate as catalyst and hydrogen peroxide,
and/or
2) a mixture of
a) at least one mineral acid,
b) hydrogen peroxide,
c) at least one metal tungstate.

2. Process according to Claim 1,
**characterized in that** the oxidation is performed at temperatures of 30-120°C, preferably in the range between 50-80°C.

3. Process according to at least one of the preceding claims,
**characterized in that** the ratio of β-isophorone to H₂O₂ is 1:0.7 - 1:3 wt%, preferably 1:1 - 1:2.2 wt%.

4. Process according to at least one of the preceding claims,
**characterized in that** the ratio of organic phase to aqueous phase varies from 1:10 to 5:1 wt%, preferably 1:4 - 2:1 wt%.

5. Process according to at least one of the preceding claims,
**characterized in that** the H₂O₂ concentration in the aqueous phase is 1-50 wt%, preferably 2-30 wt%.

6. Process according to at least one of the preceding claims,
**characterized in that** the molar ratio of catalyst to H₂O₂ is 1:10 - 1:400 wt%, preferably 1:50 - 1:200 wt%.

7. Process according to at least one of the preceding claims,
**characterized in that** quaternary ammonium salts, tertiary amines or quaternary phosphonium salts, alone or in mixtures, are employed as the phase transfer reagent.

8. Process according to Claim 7,
**characterized in that** dodecyltrimethylammonium salts, hexadecyltrimethylammonium salts, octadecyltrimethylammonium salts, methyltributylammonium salts, methyltrioctylammonium salts, dodecyldimethylamine, hexadecyldimethylamine, octadecyldimethylamine, tributylamine, trioctylamine, alkyl- , benzyl- and phenylphosphonium salts, preferably di-tert-butylmethylphosphonium tetrafluoroborate, benzyltriphenylphosphonium chloride and triphenylbutylphosphonium chloride, alone or in mixtures, are employed as the phase transfer reagent.

9. Process according to at least one of the preceding claims,
**characterized in that** phosphoric acid, phosphorous acid, polyphosphoric acid, pyrophosphoric acid, alone or in mixtures, are employed as the mineral acids.

10. Process according to at least one of the preceding claims,
**characterized in that** sodium tungstate or potassium tungstate, alone or in mixtures, is employed.

11. Process according to at least one of the preceding claims,
**characterized in that** the employed catalyst system consists of sodium tungstate, phosphoric acid and a phase transfer reagent.

12. Process according to at least one of the preceding claims,
**characterized in that** trioctylamine is employed as the phase transfer reagent.

## Revendications

1. Procédé de fabrication de céto-isophorone par oxydation catalytique de β-isophorone en présence d'au moins un catalyseur et de peroxyde d'hydrogène par une synthèse monotope, l'oxydation ayant lieu en présence d'au moins un réactif de transfert de phases dans un système biphasé, constitué par :
A) au moins une phase organique
et
B) au moins une phase aqueuse,
le système biphasé contenant :
1) au moins un polyoxométallate de tungstène en tant que catalyseur et du peroxyde d'hydrogène,
et/ou
2) un mélange de
a) au moins un acide minéral,
b) du peroxyde d'hydrogène,
c) au moins un tungstate de métal.

2. Procédé selon la revendication 1, **caractérisé en ce que** l'oxydation est réalisée à des températures de 30 à 120 °C, de préférence dans la plage comprise entre 50 et 80 °C.

3. Procédé selon au moins l'une quelconque des revendications précédentes, **caractérisé en ce que** le rapport entre la β-isophorone et H₂O₂ est de 1:0,7 à 1:3 % en poids, de préférence de 1:1 à 1:2,2 % en poids.

4. Procédé selon au moins l'une quelconque des revendications précédentes, **caractérisé en ce que** le rapport entre la phase organique et la phase aqueuse varie de 1:10 à 5:1 % en poids, de préférence de 1:4 à 2:1 % en poids.

5. Procédé selon au moins l'une quelconque des revendications précédentes, **caractérisé en ce que** la concentration d'H₂O₂ dans la phase aqueuse est de 1 à 50 % en poids, de préférence de 2 à 30 % en poids.

6. Procédé selon au moins l'une quelconque des revendications précédentes, **caractérisé en ce que** le rapport molaire entre le catalyseur et H₂O₂ est de 1:10 à 1:400 % en poids, de préférence de 1:50 à 1:200 % en poids.

7. Procédé selon au moins l'une quelconque des revendications précédentes, **caractérisé en ce que** des sels d'ammonium quaternaires, des amines tertiaires ou des sels de phosphonium quaternaires, seuls ou en mélanges, sont utilisés en tant que réactif de transfert de phases.

8. Procédé selon la revendication 7, **caractérisé en ce que** des sels de dodécyltriméthylammonium, des sels d'hexadécyltriméthylammonium, des sels d'octadécyltriméthylammonium, des sels de méthyltributylammonium, des sels de méthyltrioctylammonium, de la dodécyldiméthylamine, de l'hexadécyldiméthylamine, de l'octadécyldiméthylamine, de la tributylamine, de la trioctylamine, des sels d'alkyl-, de benzyl- et de phénylphosphonium, de préférence du tétrafluoroborate de di-tert-butylméthylphosphonium, du chlorure de benzyltriphénylphosphonium et du chlorure de triphénylbutylphosphonium, seuls ou en mélanges, sont utilisés en tant que réactif de transfert de phases.

9. Procédé selon au moins l'une quelconque des revendications précédentes, **caractérisé en ce que** l'acide phosphorique, l'acide phosphoreux, l'acide polyphosphorique, l'acide pyrophosphorique, seuls ou en mélanges, sont utilisés en tant qu'acides minéraux.

10. Procédé selon au moins l'une quelconque des revendications précédentes, **caractérisé en ce que** le tungstate de sodium ou de potassium, seul ou en mélanges, est utilisé.

11. Procédé selon au moins l'une quelconque des revendications précédentes, **caractérisé en ce que** le système catalytique utilisé est constitué par du tungstate de sodium, de l'acide phosphorique et un réactif de transfert de phases.

12. Procédé selon au moins l'une quelconque des revendications précédentes, **caractérisé en ce que** de la trioctylamine est utilisée en tant que réactif de transfert de phases.
